# EUROPEAN PATENT SPECIFICATION

(11) **EP 1 610 815 B1**
(45) Date of publication and mention of the grant of the patent: **06.01.2010**
(21) Application number: 04722902.6
(22) Date of filing: 24.03.2004
(51) Int. Cl.: A61K 39/02, A61P 31/04

(54) **FRANCISELLA STRAIN FOR LIVE VACCINE**
FRANCISELLA-STAMM FÜR LEBENDEN IMPFSTOFF
SOUCHE DE Francisella pour VACCIN VIVANT

(30) Priority: 27.03.2003 GB 0307089
(43) Date of publication of application: 04.01.2006
(73) Proprietor: THE SECRETARY OF STATE FOR DEFENCE, Salisbury Wiltshire SP4 0JQ (GB)
(72) Inventor: MICHELL, Stephen Lloyd, DSTL, Salisbury, Wiltshire SP4 0JQ (GB); OYSTON, Petra Claire Farquhar, DSTL, Salisbury, Wiltshire SP4 0JQ (GB); QUARRY, Janine Elizabeth, DSTL, Salisbury, Wiltshire SP4 0JQ (GB); TITBALL, Richard William, DSTL, Salisbury, Wiltshire SP4 0JQ (GB)
(74) Representative: Beckham, Robert William
(86) International application number: PCT/GB2004/001264
(87) International publication number: WO 2004/084935

(56) References cited:
- KARLSSON J ET AL: "Sequencing of the Francisella tularensis strain Schu 4 genome reveals the shikimate and purine metabolic pathways, targets for the construction of a rationally attenuated auxotrophic vaccine" MICROBIAL AND COMPARATIVE GENOMICS, vol. 5, no. 1, 2000, pages 25-39, XP009018156 ISSN: 1090-6592
- ELLIS J ET AL: "Tularemia" CLINICAL MICROBIOLOGY REVIEWS. OCT 2002, vol. 15, no. 4, October 2002 (2002-10), pages 631-646, XP002295463 ISSN: 0893-8512
- TITBALL R W ET AL: "Will the enigma of Francisella tularensis virulence soon be solved?" TRENDS IN MICROBIOLOGY, vol. 11, no. 3, March 2003 (2003-03), pages 118-123, XP002295464 ISSN: 0966-842X
- GRAY C G ET AL: "The identification of five genetic loci of Francisella novicida associated with intracellular growth" FEMS MICROBIOLOGY LETTERS, vol. 215, no. 1, 24 September 2002 (2002-09-24), pages 53-56, XP002295465 ISSN: 0378-1097 cited in the application

## Description

The present invention relates to the live strains of *Francisella* species for use as prophylactic or therapeutic vaccines, to compositions containing these strains.

*Francisella tularensis* is the causative agent of tularemia and is a member of the family *Francisellaceae*. There are three species within the genus *Francisella: F. tularensis, Francisella novicida* and *Francisella philomiragia*. 16S ribosomal DNA sequence analysis has placed the genus *Francisella* as a member of the γ subclass of the proteobacteria. The *F. tularensis* species was originally divided in to two biotypes, A and B, but recently four recognisable biotypes have been proposed. *F. tularensis* subspecies *tularensis*, previously known as Type A or subspecies *nearatica*, is recognised as the most virulent. It is responsible for human cases in North America and Europe and causes severe disease in mammals, especially rabbits. *F. tularensis* subspecies *palaearctica*, also known as *holartica* or Type B, is found in Europe, Asia and North America and is less virulent in humans than *F. tularensis* subspecies *tularensis. F. tularensis* subspecies *mediaasiatica* has been isolated from central Asia and subspecies *palaearctica japonica* is found only in Japan.

*F. tularensis* subspecies *philomiragia* was originally known as the "Philomiragia" bacterium before being renamed *Yersinia philomiragia*. It was finally placed in the *Francisella* genus on the basis of biochemical tests and cellular fatty acid analysis. Although *F. tularensis* subspecies *novicida* and *F. tularensis* subspecies *philomiragia* are considered pathogenic to humans they pose only a small risk.

*F. novicida* was classified into the genus *Pasteurella* in 1955, but then reclassified in 1959 into the genus *Francisella.* It was initially considered a separate species to *F. tularensis*, however recently it has been proposed that it should be designated *F. tularensis* subspecies *novicida* because of the similarities between the two species. Both of these designations are utilised herein.

At the genetic level this similarity to *F. tularensis* is greater than 99% and the two species are chemically and antigenically very similar, demonstrating strong serological cross-reactivity. However the applicants have found that *F. novicida* can be differentiated from *F. tularensis* on the basis of less fastidious growth requirements and the ability to produce acid from sucrose. *F. novicida* is fully virulent in the mouse model with a LD50 of 1.76cfu, but has reduced virulence in humans compared to *F. tularensis*.

Human cases of tularemia usually result from a bite from a vector such as biting flies, ticks and mosquitoes that have recently fed on an infected animal. However, there have been reported cases of infections caused by contact with dead animals, infectious aerosols, and ingestion of contaminated food and water. Hunters, veterinarians, walkers and farmers are at the greatest risk of contracting tularemia because they are likely to come into contact with infected animals. The incidence of tularemia in humans is usually low, but an increase in the number of cases is observed when there is an epidemic in the local animal reservoir.

In the 1940s there were attempts to make killed vaccines against tularemia consisting of whole killed cells or cell extracts, however these failed to give protection against challenge with fully virulent strains. Therefore efforts were concentrated on the production of a live vaccine. Live attenuated strains were developed in the former Soviet Union by repeatedly passaging the bacterium on media containing antiserum. Several strains were suitably attenuated for use as a vaccine and were used as such, either alone or in a mixed culture vaccine.

In 1956 a mixture of strains of *Francisella tularensis* were transferred from the former Soviet Union to the United States. From these a suitably attenuated strain was isolated and tested for safety and efficacy and was designated *F. tularensis* live vaccine strain (LVS). The vaccine is delivered via the scarification route using a dose of 0.06 ml and is followed by yearly boosters. Retrospective studies on the efficacy of the LVS vaccine based on laboratory acquired infections have shown that it affords good but not complete protection against typhoidal tularemia leading to a dramatic decease in cases. However, the incidence of the ulceroglandular form has not decreased but there appears to be a reduction in the severity of the clinical symptoms. Studies using *F. tularensis* LVS have shown that it is cell-mediated immunity that correlates with protection. Protein antigens on the surface of the bacterium induce the cell-mediated response however a large number of antigens appear to be important because there is no bias in the response towards one particular antigen. It has been found that the cytokines interleukin-1 and interferon-γ are important in providing resistance to infection. The humoral response induced by carbohydrate antigens on the bacterium also have a role in protection but can only protect against challenge by strains with reduced virulence.

The LVS vaccine is not registered and was only used to vaccinate at-risk personnel under special license, however these licenses have now been withdrawn. The main reason for this is that the genetic changes responsible for the attenuating phenotype are not understood at the molecular level. Therefore there is the possibility that the vaccine strain could revert back to the fully virulent form. A new live attenuated vaccine is therefore required.

The applicants have found that by modifying strains of *Francisella* in a particular way, useful vaccine strains can be produced.

The present invention therefore provides a strain of *Francisella* species wherein the purF or purD gene in the purine pathway has been inactivated, and which is able to produce a protective immune response in an animal, for use as a live prophylactic or therapeutic vaccine against infection by said *Francisella* species.

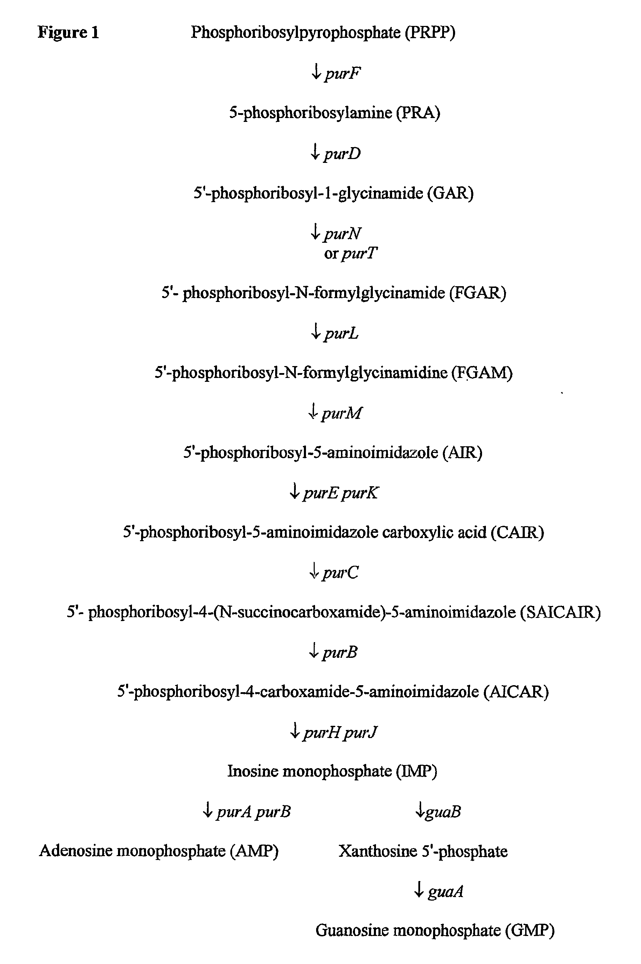

The purine pathway and enzymes that are active in it are listed in Figure 1 hereinafter. Determination of those of the genes which, if inactivated, still produce a protective immune response but do not result in a full-blown infection with virus can be determined by transforming cells using recombinant DNA *technology*, and testing the resultant transformants in animals.

The applicants have found that in order to obtain strains which are attenuated and so do not cause disease, but also are protective, it is preferable to inactivate one or more genes that encode enzymes, which are active early in the pathway. By "early", is meant enzymes which are active before the pathway branches, namely purF, purD, purN, PurT, purL, purM, purE, purK, purC, purB, purH and purJ.

For instance, inactivation of the purD gene may be effective. Mutating the purD gene has been shown to cause attenuation in other bacteria such as *Mycobacterium tuberculosis* and *S. enterica* serovar Typhimurium.

Preferably, purF or purD, the first two enzymes in the pathway may be inactivated.

In particular, the applicants have found that inactivation of the *purF* gene produces a live vaccine strain. As illustrated hereinafter, a strain of *F. novicida* with an inactivated *purF* gene is attenuated in vivo when compared to a wild-type strain. It has been found to be able to survive and replicate in macrophages and it is suggested that the levels at which this occurs are sufficient to stimulate a immune response before it is cleared by the immune system. This strain has found to be protective in mice and so may give rise to a live vaccine.

Suitably the gene is not *purA*. This gene converts inosine monophosphate to adenosine monophosphate (AMP) and its inactivation produces a requirement for adenine. A *purA* mutant has been produced in other pathogens, in particular *Salmonella enterica* serovar *typhimurium* and *Edwardsiella ictaluri* and these have proven to be attenuated, and in some cases also protective. As illustrated hereinafter however, the applicants have found that although inactivation of this gene in *F. novicida* severely affects it growth in vitro and causes a high degree of attenuation in vivo, it was not adequately protective of mice against subsequent challenge with *F. novicida*. It is possible that the inability of the *purA* mutant to survive in macrophages means that it is unable to colonise the mouse cells for a sufficient period of time to stimulate a protective immune response.

The strain of the invention suitably has a further defined mutation so as to reduce the risk of the bacterium reverting to a virulent form. In this case, the mutation is in a gene which is selected so that the strain is suitably attenuated, but can still retain the ability to stimulate a sufficient immune response to provide long term protection. Suitable additional mutations can be identified using conventional methods, and examination and analysis of the current live vaccine strain (LVS) may assist in the identification, since this strain has demonstrated that protection can be achieved using an attenuated strain.

Gene inactivation can be carried out using any of the conventional methods. Typically, the strain is transformed with a vector which has the effect of downregulating or otherwise inactivating the gene. This can be done by mutating control elements such as promoters and the like which control gene expression, by mutating the coding region of gene so that any product expressed is inactive, or by deleting the gene entirely. Alternatively, the gene can be inactivated at the RNA or protein level, by transforming the cell so that it expresses a sense or anti-sense construct which binds to DNA or RNA encoding the gene to prevent transcription thereof.

It may also be possible to inactivate the product at the protein level, by causing the cell to express a protein which binds to and inactivates the enzyme.

Preferably however, the gene is inactivated by complete or partial deletion mutation or by insertional mutation.

Transformation can be carried out in a variety of ways. It has been reported in the literature that F. novicida U112 can be transformed by electroporation and chemically. The applicants have found however that transformation is suitably carried out using cryotransformation, as illustrated hereinafter. Crytransformation has been used to produce an isogenic defined *purA* mutant in *F. novicida*, showing not only that the particular construct can undergo homologous recombination in a *Francisella* host, but also that *F. novicida* is amenable to cryotransformation. In this method, cells are mixed with transformation buffer and plasmid DNA, and then frozen for a period, for example in liquid nitrogen. Recovered cells are incubated and then cultured and selected using conventional methods.

In particular, the strain used is a *Francisella tularensis, such as F. tularensis* subspecies *novicida* or *F. tularensis* subspecies *tularensis*. Preferably the strain is a strain of *Francisella tularensis* subspecies *tularensis*.

These strains provide useful vaccines. They are therefore preferably formulated into pharmaceutical compositions, in which they are combined with a pharmaceutically acceptable carrier. These form a further aspect of the invention.

Suitable carriers may be solid or liquid carriers as is understood in the art. They may suitably be formulated for administration to mucosal surfaces (for example for oral use, of for administration by inhalation or insufflation) or for parenteral administration.

In particular they are formulated as sterile aqueous or oily solutions for intravenous, subcutaneous, intramuscular or intramuscular dosing.

Alternatively they are formulated for administration to mucosal surfaces and in particular for intranasal application.

Compositions are suitably prepared in unit dosage forms, as conventional in the art. They are administered at dosages which are determined using clinical practice, and depend upon factors such as the nature of the patient, the severity of the condition, and the precise vaccine strain being employed. Typically dosage units will comprise 10⁵ - 10⁸ cfu. Dosages may be boosted as appropriate or necessary.

Compositions may also contain further immunogenic reagents which are effective against *F. tularensis* or other diseases. They may further contain other agents such as adjuvants and the like, which enhance the host's immune response to the vaccine.

In a further aspect the invention provides the use of a strain of *Francisella* species wherein the purF or purD gene has been inactivated, and which is able to produce a protective immune response in an animal, in the preparation of a live prophylactic or therapeutic vaccine against infection by said *Francisella* species.

Prevention or treatment of infection by a *Francisella* species can be effected by administering to an animal an effective amount of a live strain as described above, or a composition as described above.

In particular, the method is used to protect or treat infection by *Francisella tularensis* subspecies *tularensis*.

Novel strains which are suitable for vaccine use form a further aspect of the invention. In particular, the invention provides a strain of Francisella tularensis subspecies tularensis wherein a gene that encodes a *purF* or purD gene has been inactivated by complete or partial deletion mutation.

The invention will now be particularly described by way of Example with reference to the accompanying diagrammatic drawings in which:
Figure 1 shows the de novo purine pathway;

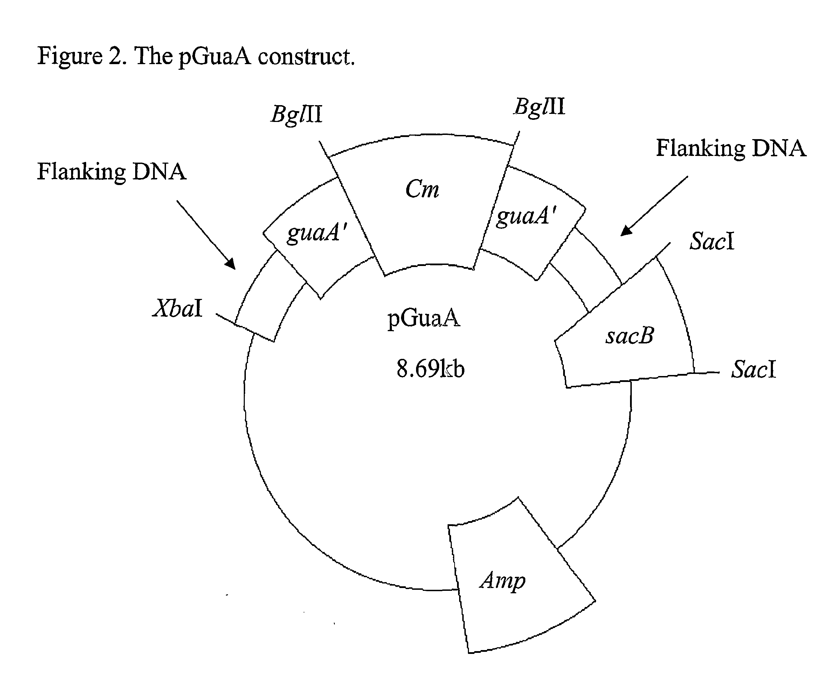

Figure 2 illustrates a construct used in the examples, the pUC18 guaA' construct;

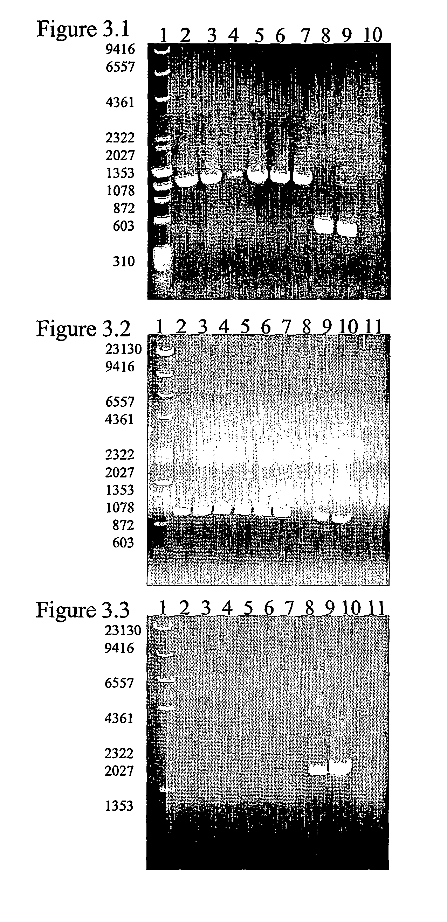

Figure 3.1 shows the results of a PCR to amplify the purA gene, using the screen F and screen R primers, wherein lane 1: shows molecular weight markers (DRIgest III) Roche, size shown in base pairs), lane 2:*F. novicida* purA 1, lane 3:*F. novicida* purA 2, lane 4:*F*. *novicida* purA 3, lane 5:*F. novicida* purA 6, lane 6:*F. novicida* purA 7, lane 7:*F. novicida* purA 25, lane 8:*F. novicida* U112, lane 9:*F*. *tularensis* HN63 purA integrant 15, lane 10: control- dH₂O.
Figure 3.2 shows the results of a PCR to amplify the chloramphenicol cassette, using the CAM BamF and CAM BamR primers, wherein lane 1: shows molecular weight markers (DRIgest III) Roche, size shown in base pairs), lane 2:*F*. *novicida* purA 1, lane 3:*F. novicida* purA 2, lane 4:*F. novicida* purA 3, lane 5:*F*. *novicida* purA 6, lane6:*F*. *novicida* purA 7, lane 7:*F*. *novicida* purA 25, lane B:*F*. *novicida* U112, lane 9:*F. tularensis* HN63 purA integrant 15, lane 10: pGEM CAM, lane 11: control-dH₂O.
Figure 3.3 shows the results of a PCR to amplify the *sacB* gene, using the SacBFor and sacBRev primers, wherein lane 1: shows molecular weight markers (DRIgest III) Roche, size shown in base pairs), lane 2:*F. novicida* purA 1, lane 3:*F. novicida* purA 2, lane 4:*F. novicida* purA 3, lane 5:*F. novicida* purA 6, lane 6:*F. novicida* purA 7, lane 7:*F. novicida* purA 25, lane 8:*F. novicida* U112, lane 9:*F. tularensis* HN63 purA integrant 15, lane 10:pGEM *sacB*, lane 11: control- dH₂O.

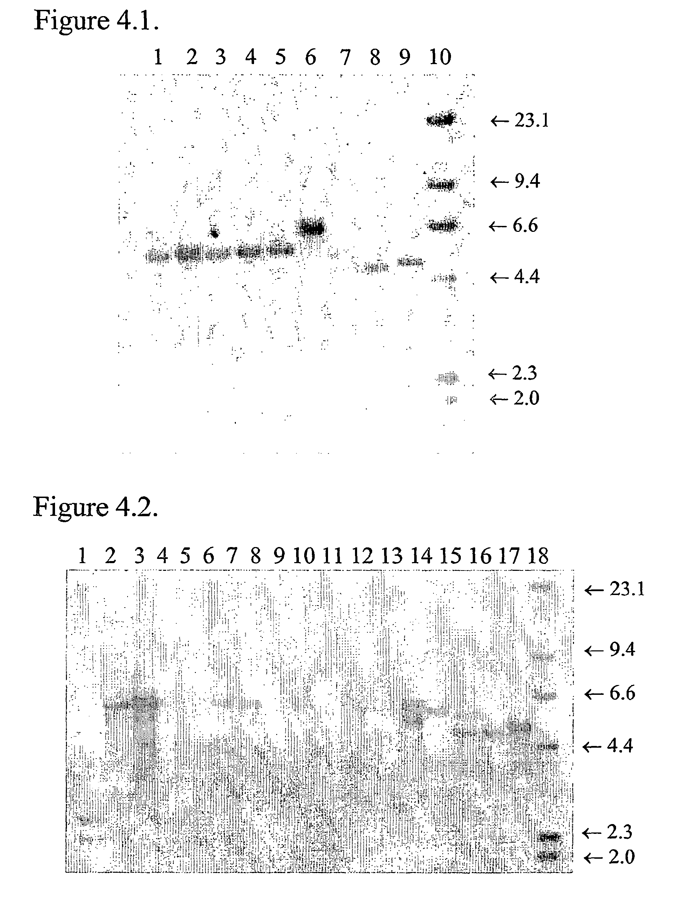

Figure 4.1 shows a Southern blot of *F. novicida* and *F.tularensis* DNA probed for the purA gene after digestion with MluI, wherein lane 1: shows the results for *F. novicida* purA 1, lane 2:*F. novicida* purA 2, lane 3:*F. novicida* puA 3, lane 4:*F. novicida* purA 6, lane 5:*F. novicida* purA 7, lane 6:*F. novicida* purA 25, lane 7: *F. tularensis* HN63 purA integrant 15, lane 8 *F. tularensis* Schu4, lane 9: *F. novicida* U112, lane 10: Molecular size marker, sizes indicated in kilobase pairs.
Figure 4.2 shows a Southern blot of *F. novicida* and *F.tularensis* DNA probed for the purA gene after digestion with MluI, wherein lane 1: shows the results for Molecular size marker, sizes indicated in kilobase pairs, lane 2:*F. novicida* purA 2.1, lane *3:F. novicida* purA 2.2, lane 4:*F. novicida* purA 2.3, lane 5:*F. novicida* purA 2.4, lane 6:*F*. *novicida* purA 2.5, lane 7:*F. novicida* purA 3.1, lane 8:*F. novicida* purA 3.2, lane 9:*F. novicida* purA 3.3, lane 10:*F. novicida* purA 3.4, lane 11:*F. novicida* purA 3.5, lane 12:*F. novicida* purA 3.6, lane 13:*F. novicida* purA 3.7, lane 14:*F. novicida* purA 3.8, lane 15: *F. tularensis* HN63 purA integrant 15, lane 16 *F. tularensis* Schu4, lane 17: *F. novicida* U112, lane 18: Molecular size marker, sizes indicated in kilobase pairs.

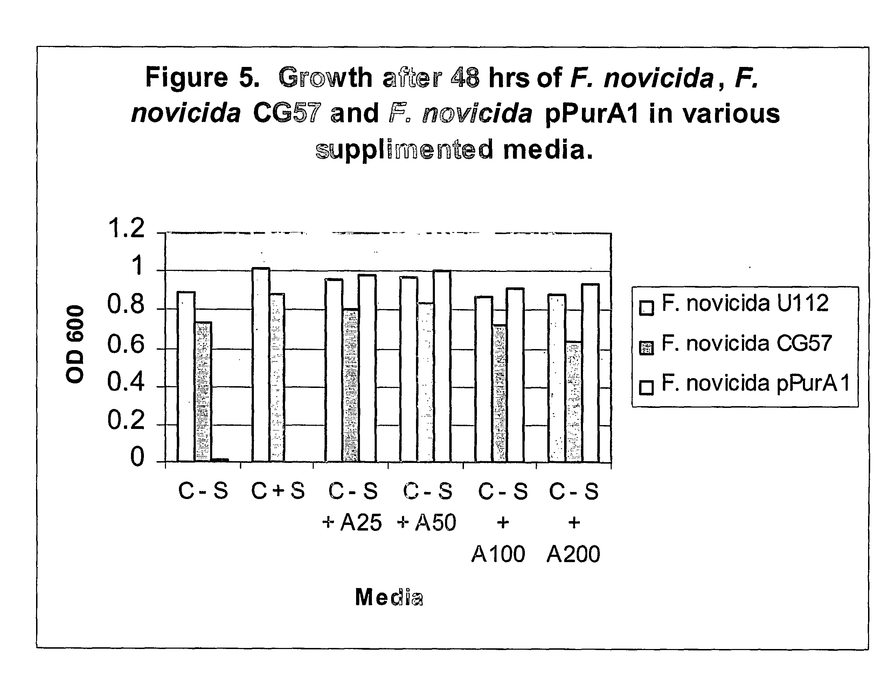

Figure 5 shows the growth after 48 hours of F. novicida, F novicida CG57 and F. novicida purA 1 in various supplemented media.

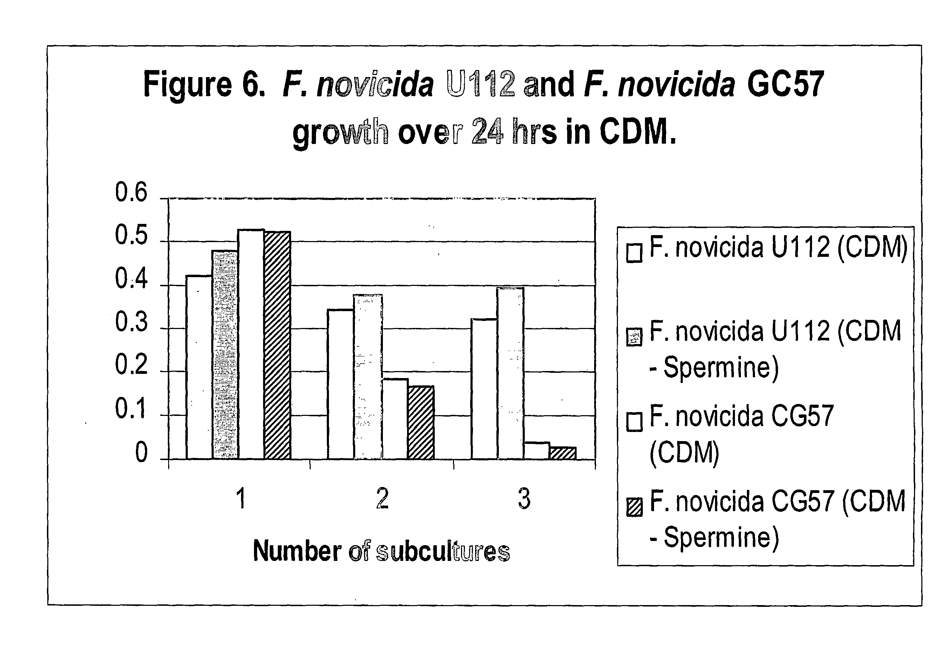

Figure 6 is a graph showing F. novicida U112 and F. novicida GC57 growth over 24 hours in CDM.

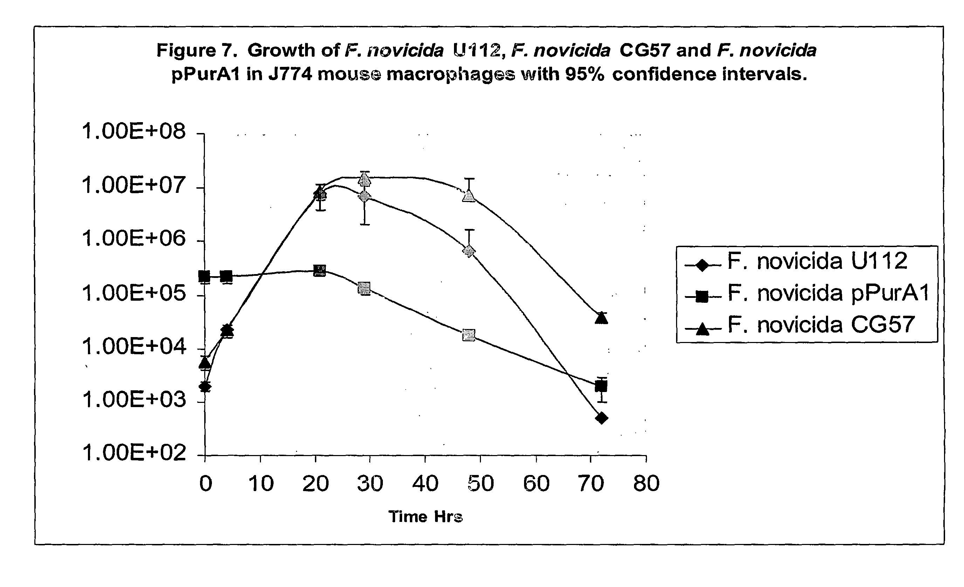

Figure 7 illustrates the growth of F. novicida, F novicida CG57 and F. novicida purA 1 in J774 mouse macrophages with 95% confidence intervals.

### Example 1

### Preparation of transformed strains

### Materials and Methods

### Bacterial Strains

**Table 1**

| Strain | Details | Source/Supplier |
|---|---|---|
| *E. coli* DH5α | F'/endAl hsdR17 (rₖ⁻ mₖ⁺) | Life Technologies |
| | Sup E44 thi-1 recA1 gyrA | |
| | (nalR) Δ(thyA) 57 | |
| | hsdS3 (rₖ⁻mₖ⁺) | |
| *F. novicida* U112 | Wildtype | ATCC |
| *F. novicida* CG57 | TnMax2. Em^{R} | Dr F Nano |
| *F. novicida* CG57::GFP | TnMax2. Em^{R} | see hereinafter |
| *F. novicida* purA | ΔpurA. Cm^{R} | see hereinafter |

### Plasmids

**Table 2**

| Plasmid | Details | Source |
|---|---|---|
| pKK202 | Tc^{R}Cm^{R} | F.O.I. Sweden {Norqvist, 1996 #1} |
| pUC18 | Amp^{R} | New England Biolabs |
| pUC18 purA' | ΔpurA. Cm^{R} | |
| pUC18 guaA' | ΔguaA. Cm^{R} | see hereinafter |

The pUC18 guaA' plasmid was constructed by amplifying two sections of the *gua* gene plus flanking regions by PCR. The primer pairs GUA2For/GuaABg1R and GuaA3Rev/RevABg1F were used in the amplification. The two fragments were then cloned into pUC18 on the XbaI and SacI sites. A chloramphenicol cassette, amplified by PCR using the primers CAM Bg1F and CAM Bg1II, was inserted between the two sections of the gene on an engineered BglII site (Table 3). The *sacB* gene from the plasmid CVD422 was cloned into the pUC18 backbone on the SacI restriction site (Figure 2).

Regions of the *purA* gene were amplified from *F. novicida* U112 template DNA and sequenced. The sequenced regions showed a 91-98% identity to the *F. tularensis* strain SCHU4 genome sequence. A *purA* construct was made using the same strategy as described above for the *guaA* construct.

### Growth conditions

*E. coli* strains were grown in Luria-Bertani (LB) media at 37°C. *F. novicida* was grown on supplemented blood cysteine glucose agar, in Chamberlains defined media, Tryptone soya broth containing 0.1% cysteine or in MCPH broth containing 2% glucose. Selective media was supplemented with antibiotics to the following concentrations: ampicillin at 55µg/ml, chloramphenicol at 30µg/ml, tetracycline 13.5µg/ml.

### Freezes stocks

For *E. coli* strains, 100% glycerol was added to overnight cultures to give a final concentration of 30% before storage at -70°C.

*F. novicida* cells were harvested from two spread plates and resuspended in a 1ml volume of 30% glycerol in phosphate buffered saline.

### Restriction digests

Plasmid digests were set up containing 2µl of plasmid DNA, 1µl of each enzyme required, 1µl of a compatable buffer and the volume made up to 10µl with dH₂O. Digests were incubated at 37°C overnight.

### Agarose gel electrophoresis

PCR products and digests were separated on a 0.7% (wt/vol) agarose-TAE (Tris-acetate EDTA) gel containing 0.5µg/ml ethidium bromide per ml and run against DRIgest III markers (Amersham Pharmacia Biotech).

### Primers and Polymerase chain reaction

The gene fragments were amplified from *F. novicida* DNA using the polymerase chain reaction (PCR) using 30 cycles at 94°C (30 s), 55°C (30 s), 72°C (60 s) followed by 72°C (10 min) and 4°C. The Perkin Elmer 9600 GeneAmp PCR system was used. Primers used are shown in Table 3.

**Table 3**

| Primer | Sequence 5' to 3' | No* |
|---|---|---|
| CAM BamF | CGC GGA TCC GTA AGA GGT TCC AAC TTT CAC | 1 |
| CAM BamR | CGC GGA TCC TTA CGC CCC GCC CTG CCA CTC ATC | 2 |
| sacBFor | CGA TCT CAA GAA GCA GAC CGC TAA CAC A | 3 |
| sacBRev | CGA GCT CAT AGT TCA TAT GGG ATT CAC C | 4 |
| screen F | GTA GAA TTC GTA GGT GTG GTT GGT TAG | 5 |
| screen R | GTA ACT TGC TGT CCT GAA TAG TCT TGA | 6 |
| GuaA2For | TGC TCT AGA TAT AGC TAT TGC CGT AGG AAT | 7 |
| GuaABglR | CGT GCA AGA TCT GCC ATT TAG CAT TCT CTA | 8 |
| GuaA3Rev | CGA GCT CCA GCG CCA ATA CCA GCA CCA | 9 |
| GuaABglF | GCA CGT AGA TCT AGC GTA GAT ATG AGT ATG | 10 |

| | | |
|---|---|---|
| *SEQ ID NO | | |

### Isolation of plasmid DNA

Plasmid DNA was isolated using the Qiagen mini and maxi plasmid isolation kits (Basingstoke, UK) as detailed in the manufacturer's instructions.

### Genomic DNA extractions

*F. novicida* U112 and *F. novicida* purA mutant DNA was isolated using the Puregene DNA Isolation kit (Gentra systems) according to the manufacturer's instructions. The DNA was resuspended in 50µl of DNA hydration solution and incubated overnight at room temperature. The DNA was stored at -20°C.

### Transformation methods

To establish an optimal transformation method, the efficiency of transforming F. novicida U112 using a range of methods was investigated using the stably replicating shuttle plasmid pKK202. Cryotransformation was found to be the most effective technique.

### Cryotransformation

The cells from two *F. novicida* U112 spread plates were harvested and resuspended in 400µl 0.2M KCL of pipetting. A 25µl vlume of bacterial suspension were mixed with 25µl of transformation buffer. For each positive sample 5µl of plasmid DNA were added and for the negative control 5µl of dH₂O. The samples were incubated at room temperature for 10 min before being frozen for 5 min by immersion in liquid nitrogen. The samples were removed from the liquid nitrogen and incubated at 37°C for 5 min.

The cells were spotted onto a supplemented BCGA plate and incubated at 37°C for 4 hr. The cells were harvested from the plate and resuspended by pipetting in 400µl of 0.2M KCl. Aliquots of 100µl were plated onto supplemented BCGA chloramphenicol plates. For pKK202, 100µl aliquots were also plated onto supplemented BCGA tetracycline plates. The plates were incubated at 37°C.

This cryotransformation of *F. novicida* with the plasmid pKK202 yielded transformants at a frequency of 5.8x10⁻¹ per ng DNA. The method was also effective for the transformation of pKK202 into F.tularenis HN63.

### Competent F. novicida cells for electroporation

*F. novicida* was grown in TSB-C with shaking at 37°C to an early to mid-exponential growth phase (OD₆₀₀nm 0.6), 40ml volumes of culture were spun at 12000g for 10 min and then the pellet resuspended in 40ml 500mM sucrose. This washing step was repeated and then the pellet was resuspended in 10ml 500mM sucrose. After a 4 min centrifugation step at 12000g the pellet was resuspended in 160µl of 500mM sucrose. Glycerol competent cells were made by washing the cells in 10% glycerol using the method described above.

### Electroporation

For each electroporation 40µl aliquots of cell suspension were added to a 0.2cm gap electroporation cuvette. A total of 200ng of plasmid DNA in a 3µl volume of dH₂O was added prior to electroproation. Electroporation was conducted using a Gene Pulser (Bio-Rad) at a voltage of 1.5kV, a capacitance of 25µF and a resistance setting of 200Ω.

### Southern blotting

For each digest 2-3µg of DNA, 15 U of restriction enzyme (NsiI or MluI (Roche)) and 1.5µl of a compatable buffer were used in a final volume of 15µl. The digests were incubated at 37°C overnight. The digested DNA was separated on a 0.7% agarose gel, dig labelled markers were included on the gel as standards (DNA molecular weight marker II DIG-labelled, Roche).

After the gel had run it was washed in denaturing solution for 45 min. It was then transferred into neutralising buffer for 30 min followed by a second wash for 15 min in fresh buffer. Capillary action using 10X SSC was used to transfer the DNA from the gel to a positively charged nylon membrane overnight. The membrane was air dried for 30m in and then baked at 120°C for 30 min.

The membrane was prehybridised by incubating the membrane at 37°C for 1hr in DIG-Easy Hyb solution (Sigma).

The purA probe was synthesised by PCR with the primers purABamF and purARev (Table 3) using DIG labelled dNTPs. For hybridisation, 2500µg of probe was boiled for 8min and placed on ice, this was added to 100ml DIG Easy Hyb solution (Sigma) and the membrane was incubated in this overnight at 37°C.

The membrane was washed twice with 2XSSC+0.1% SDS for 5min at room temperature and twice with 0.1XSSC+0.1% SDS at 68°C for 15min.

The membrane was then rinsed for 3 min in 1x wash buffer (Malic acid buffer + 3% Tween 20) and then incubated in 1.5x blocking solution for 45min at room temperature. (Blocking solution - DIG block + 1X malic acid buffer.)

The block was removed and replaced with fresh 1.5x blocking solution containing antidigoxigenin-AP Fab fragment antibody at a dilution of 1:10,000. This was incubated for 30min at room temperature. The membrane was washed twice for 15 min in 1x wash buffer at room temperature then allowed to equilibrate in detection buffer.

Approximately 20 drops of the substrate diosodium 3-(4-methoxyspiro {1,2-dioxentane-3,2'-(5'chloro)tricyclo-[3.3.1.1^{3,7}]decan}-4-yl) phenyl phosphate (cspd) was applied to a transparent polyethylene folder. The membrane was placed on the plastic and a further 20 drops of cspd substrate applied to the membrane. The remaining plastic was folded over and the membrane and was incubate for 15 min at room temperature and then for a further 15 min at 37°C.

The membrane was exposed to Lumi-film chemiluminescent detection film. The film was then developed in developing solution (Kodak) and then fixed in fixing solution (Kodak). It was then washed in water and allowed to dry.

### Macrophage assay

Bacterial cells from half a spread plate were resuspended in 3ml L-15 containing Glutamax and 10% final conc. foetal calf serum (Life Technologies). The spectrophotometer reading of this suspension was taken at an optical density of 600nm. This suspension was suitably diluted to give an approximate concentration of 1x10⁹cfu/ml. An aliquot of this suspension was serially diluted and plated onto BCGA + supps plated, which were incubated at 37°C to determine the viable count.

The L-15 was aspirated from each well and 100µl of suspension overlaid onto the macrophages. This was repeated for 6 plates (each with 2 wells), plus a control well. Each well contained 1 x 10⁸ macrophages and therefore each monolayer was infected with a multiplicity of infection of 10. The cells were incubated for 55 min at 37°C. The media was aspirated and each well was overlaid with L-15 containing 10µg/ml gentamycin. The cells were incubated at 37°C for 1 hr. The media was then removed and replaced with L-15 containing 2µg/ml gentamycin. This was T0. At this timepoint two wells for each bacterial strain were aspirated and the macrophages washed twice with PBS, 1ml of dH₂O then added to each well. The macrophages were then incubated at room temperature for 5 min before they were disrupted by pipetting thirty times. The cell lysate was then serially diluted in PBS and suitable dilutions were plated onto BCGA + supp plates, which were incubated at 37°C for two days. Further time points were taken at 4, 21, 29, 48 and 72 hours.

### Results

### Recovery of F. novicida pUC18 purA transformants

*F. novicida* purA transformants were recovered on selective media after three separate cryotransformations. Using the PCR primer pair screenF and screenR two of the transformants were identified as integrants whereas the other thirty six clones appeared to be defined mutants (Figure 3.1). A PCR, using the primers sacBFor and sacBRev which aplify the *sacB* gene, confirmed the presence of the gene from integrant DNA but its absence from mutant DNA (Figure 3.2). For all the clones that were screened the chloramphenicol gene was successfuly amplified using the PCR primers CAMBamF and CAMBamR (Figure 3.3).

### Southern blotting

Genomic DNA from *F. novicida* purA mutants 1, 2, 3, 6, 7, 25, 2.1-2.5, 3.1-3.8, *F. tularensis* integrant 15, *F. tularensis* SCHU4 and *F. novicida* was digested with the restriction endonuclease *MluI*. The digest was analysed by Southern blotting and probed for the *purA* gene. The probed fragment was the same size for all the mutants and showed the size increase in comparison to *F. novicida* corresponding to the chloramphenicol cassette (Figures 4.1, 4.2). The southern blot was repeated using the restriction enzyme *NsiI* (Data not shown). These results also confirmed the presence of the chloramphenicol cassette.

It was therefore established that integrants and mutants could be differentiated on the basis of PCR and southern blotting results.

### Selection on sucrose

The *sacB* gene had been incorporated into the construct as a counterselectable marker so that once integration of the plasmid had occurred double crossover mutants could be selected for on sucrose. However the majority of the transformants obtained has lost the *sacB* gene without the need for selection. In the case of the two integrants these strains were grown in the presence of 5% sucrose in CDM and on BCGA-supp+10% sucrose to select for the double crossover events. Colonies that grew in the presence of sucrose were screened by PCR using the screenF and screenR primers. All the clones tested proved to be integrants and appeared to be insensitive to the sucrose. Therefore, in this case, sucrose selection was not effective. This may be due to the *sacB* gene not being expressed properly.

The *F. novicida* purA mutants were isolated without any sucrose selection, which implies that after integration of the plasmid the double crossover event spontaneously occurred. This is not unlikely because the large homologous DNA flanking regions would make plasmid in the integrated state unstable, this event has also been recorded in other species such as *M. tuberculosis.* However, this hypothesis contradicts the observations that the two integrants obtained appear to be stable and have not subsequently undergone the second crossover event. It is possible that in these clones the plasmid has integrated in a way that prevents the second crossover event occurring.

### F. novicida CG57

For comparison with *F. novicida* purA, *F. novicida* CG57 was provided by Dr. F. Nano. This strain was identified during a screen of *F. novicida* mutants generated using the transposon TnMax2 as being compromised in its ability to grow in mouse macrophages. The interrupted gene was identified as *purF*, which encodes the enzyme amidophophoribosyltransferase {Gray, 2002 FEMS Microbiology Lett 2002 Sept 24; 215(1): 53-6}. It may also be prepared using a strategy similar to that described above in reflection to the *purA* mutant. This catalyses the first step in the *de novo* synthesis of purines converting phosphoribosylpyrophosphate (PRPP) to 5-phosphoribosylamine (PRA) (Figure 1).

### Growth of F. novicida purA in vitro

Inactivation of the purA gene produces a requirement for adenine, in Chamberlains defined media (CDM) the only source of adenine was thought to be spermine. Therefore to investigate the adenine auxotrophic phenotype of *F. novicida purA*, clone 1 was grown in complete CDM and in spermine deficient CDM. It was also grown in spermine deficient CDM supplemented with varying concentrations of adenine (Figure 5). An adenine concentration of 25µg/ml was sufficient to enable *F. novicida purA 1* to grow to a level comparable with *F. novicida* U112. It was also confirmed that the other purA mutants required the provision of adenine to grow in spermine deficient CDM (Data not shown). The inclusion of *F. novicida* CG57 demonstrated that the provision of adenine only affected the growth of the purA mutant.

### Growth of F. novicida CG57 in vitro

Disruption of the purF gene leads to an auxotrophic requirement for purines therefore to characterise the phenotype of *F. novicida* CG57 it was repeated sub-cultured in CDM with and without spermine. Sub-culture inhibited the growth of the bacterium in both CDM and CDM spermine (Figure 6).

### Phenotype of F. novicida purA 1 and F. novicida CG57 in vitro

Failure of the *F. novicida* purA 1 to grow in CDM with and without spermine suggested that the strain was either unable to utilise spermine as a source of purines or that the supply of purines in CDM was insufficient to compensate for the inactivation of the *purA* gene. Provision of a source of adenine allowed the purA mutant to grow which confirmed the phenotype characteristic of the mutation.

*F. novicida* CG57 was initially able to grow in CDM without spermine however the rate of growth decreased after repeat passaging in spermine deficient CDM. This suggested that there are intracellular stores of intermediates and/or substrates that for a limited period can be utilised by the bacterium to compensate for the inactivated gene.

### Mouse macrophage assay

The growth of *F. novicida* U112, *F. novicida* CG57 and *F. novicida* purA clone 1 was studied in J774 mouse macrophages (Figure 7). The number of bacteria used to infect each well was determined (Table 4).

**Table 4**

| Strain | Number of bacteria per well |
|---|---|
| *F. novicida* U112 | 6.65 x 10⁶cfu |
| *F. novicida* purA 1 | 1.61 x 10⁸cfu |
| *F. novicida* CG57 | 1.17 x 10⁹cfu |

It was observed that the *F. novicida* purA clone 1 entered the macrophages at a 1000 fold higher rate than the wildtype but over the first 21hrs number of bacteria recovered only increased slightly. From 21 to 72 hrs the number of bacteria recovered gradually dropped and microscopic examination of the macrophages showed that the monolayer remained intact throughout the assay.

The number of bacteria recovered over the first 21 hrs for *F. novicida* U112 and *F. novicida* CG57 increased by four orders of magnitude. At subsequent time points the number of bacteria enumerated fell, however microscopy examination of the macrophages revealed that the monolayer was breaking up. These results show no difference in the rate of growth between *F. novicida* U112 and *F. novicida* CG57.

These results suggest that although *F. novicida* purA 1 is capable of infecting J774 mouse macrophages it is unable to replicate and that the infection can be cleared. It is possible that inside the macrophages the levels of adenine are insufficient to enable the bacteria to multiply. The disruption of the *purA* gene may also have a detrimental effect on other cellular functions which would usually allow the bacterium to replicate and protect itself whilst inside the macrophage. It has been reported that *Francisella* enters macrophages by a cytochalasin B independent pathway, i.e. the bacteria are able to actively invade the macrophages rather than being taken up via microfilament mediated phagocystosis. However the difference in the number of bacteria recovered at T0 between *F. novicida* U112 and *F. novicida* purA 1 implies that either the mutant is able to invade more successfully than the wildtype or that the macrophages are able to actively take up the *F. novicida* purA bacteria. This would suggest that the wildtype strain has a way of controlling its rate of invasion or uptake and that disrupting the *purA* gene has removed this control.

Although *F. novicida* CG57 grew at the same rate and to similar bacterial titres as *F. novicida* U12, twice the number of bacteria were initially used to infect the monolayer. Taking this into account *F. novicida* CG57 does appear to be slightly defective in its ability to replicate in J774 mouse macrophages. This supports the published data regarding this strain {Gray, et al. 2002 supra.}.

After 24 hrs the number of bacteria recorded at subsequent timepoints for *F. novicida* U112 and *F. novicida* CG57 begins to fall. This effect is caused by the way in which the bacteria are recovered from the assay. Replication of the bacteria in the macrophages causes them to detach from the base of the well, this was confirmed by examination of the monolayer under the light microscope. This means that washing of the monolayer removes these floating cells, therefore the number of bacteria counted only reflects those that were recovered from the monolayer. Thus the counts at the 24, 48 and 72 hr timepoints are likely to be an underestimation of the true numbers of bacteria per well. Microscopic examination of the monolayers infected with *F. novicida* purA 1 showed that even after 72 hours the monolayers were intact and looked relatively healthy.

### Example 2

### Virulence challenge in mice

Bacterial cells from two spread plates were resuspended in 3ml PBS. This suspension was serially diluted to 10⁻¹¹. Spectrophotometer readings were taken at an optical density of 600nm of dilutions 0 to 10⁻⁴. Aliquots of 100µl of dilutions 10⁻⁶-10⁻¹¹ were plated in duplicate onto BCGA + supps and incubated for 24hr at 37°C. This was repeated for *F. novicida* U112, *F. novicida* CG57 and *F. novicida* purA1. Suitable dilutions were chosen depending on the required dose. Volumes of 100µl were given interperitonealy to Balb/c mice as described below.

To determine the median lethal dose (MLD) for *F. novicida* U112, groups of 5 Balb/c mice were immunised intra-peritoneally (IP) with a range of doses of between 0.01 and 115.5 cfu. (Table 5) From these results the MLD was calculated as 1.7 cfu. This level of virulence is comparable to the MLD of 1 cfu observed for the F. tularensis type A and type B strains. Therefore although F. novicida U112 has low pathogenicity in humans as compared to F. tularensis strains, this difference in virulence is not seen in the Balb/c mouse model.

Surviving mice were challenged with 100MLDs of *F. novicida* U112. No protection was observed.

**Table 5**

| Strain immunised | Dose immunised | Number of survivors | Number of survivors after challenge with *F. novicida* U112 |
|---|---|---|---|
| *F. novicida* U112 | 0.01cfu | 5/5 | 0/5 |
| *F. novicida* U112 | 0.11cfu | 5/5 | 0/5 |
| *F. novicida* U112 | 1.15cfu | 3/5 | 0/3 |
| *F. novicida* U112 | 11.5cfu | 0/5 | - |
| *F. novicida* U112 | 115cfu | 0/5 | - |

### F. novicida purA 1

To determine the MLD for *F. novicida* purA 1, groups of 5 Balb/C mice were dosed IP with a range of doses between 0.01-155cfu. No deaths were observed. The mice were challenged with 100MLDs *F. novicida* U112 but no protection was seen in the immunised mice (Data not shown).

The dose was increased to between 3.3 x 10² -3.3 x 10⁶ cfu. No fatalities were observed so it was concluded that the MLD exceeded 3.3 x 10⁶ cfu.

The mice were challenged with 100MLDs of *F. novicida* U112 and it was shown that *F. novicida* purA 1 conferred some protection but this response was not dose dependent (Table 6). Additional dosages would enhance the protective effect.

**Table 6**

| Strain immunised | Dose immunised (cfu) | Challenge strain | Number of survivors |
|---|---|---|---|
| *F. novicida* purA 1 | 3.3 x 10² | *F. novicida* U112 | 2/5 |
| *F. novicida* purA 1 | 3.3 x 10³ | *F. novicida* U112 | 1/5 |
| *F. novicida* purA 1 | 3.3 x 10⁴ | *F. novicida* U112 | 0/5 |
| *F. novicida* purA 1 | 3.3 x 10⁵ | *F. novicida* U112 | 1/5 |
| *F. novicida* purA 1 | 3.3 x 10⁶ | *F. novicida* U112 | 1/5 |
| Controls | - | *F. novicida* U112 | 0/5 |

### F. novicida CG57

To determine the MLD for *F. novicida* CG57, groups of 5 Balb/C mice were dosed IP with a range of doses between 0.002 - 24.5. No deaths were observed.

The mice were challenged with 100MLDs of *F. novicida* U112 (Table 7). From the results was observed that a dose of 24.5cfu of *F. novicida* CG57 could confer complete protection against a *F. novicida* challenge of 100MLDs.

**Table 7**

| Strain immunised | Dose immunised | Challenge strain | Number of survivors |
|---|---|---|---|
| *F. novicida* CG57 | 0.002 cfu | *F. novicida* U112 | 0/5 |
| *F. novicida* CG57 | 0.02 cfu | *F. novicida* U112 | 0/5 |
| *F. novicida* CG57 | 0.24 cfu | *F. novicida* U112 | 2/5 |
| *F. novicida* CG57 | 2.45 cfu | *F. novicida* U112 | 3/5 |
| *F. novicida* CG57 | 24.5 cfu | *F. novicida* U112 | 5/5 |

The doses of *F. novicida* CG57 were increased to between 1.58 x 10³ - 1.58 x 10⁷ cfu. Deaths were recorded in the two groups that received the highest dose of *F. novicida* CG57 and from these results the MLD was calculated as 6.67 x 10³ cfu (Table 8). The survivors were challenged with 1000MLDs of *F. tularensis* LVS (which is lethal to mice). Survival of all the mice challenged demonstrated that *F. novicida* CG57 at a dose of 1.58 x 10³ cfu or greater could protect against a *F. tularensis* LVS challenge (Table 8).

**Table 8**

| Strain immunised | Dose immunised | Number of survivors | Survivors after challenge with *F. tularensis* LVS |
|---|---|---|---|
| *F. novicida* CG57 | 1.58 x 10³ cfu | 5/5 | 5/5 |
| *F. novicida* CG57 | 1.58 x 10⁴ cfu | 5/5 | 5/5 |
| *F. novicida* CG57 | 1.58 x 10⁵ cfu | 5/5 | 5/5 |
| *F. novicida* CG57 | 1.58 x 10⁶ cfu | 1/5 | 1/1 |
| *F. novicida* CG57 | 1.58 x 10⁷ cfu | 0/5 | - |
| Control | - | | 1/5 |

These results show that although the *F. novicida* purA' clone was highly attenuated in the Balb/c mouse model, it was not protective against a challenge of 100MLDs of *F. novicida* U112.

Although F. novicida GC57, which has an inactive *purF* gene was also defective for growth in mouse macrophages and was attenuated in the Balc/c mouse model, it was protective against a challenge of 100MLDs of F. novicida U112 and 100MLds of *F. tular*ensis LVS. This therefore indicates that such a strain could form the basis of a live attenuated vaccine.

## Claims

1. A strain of *Francisella* species wherein the purF or purD gene has been inactivated, and which is able to produce a protective immune response in an animal, for use as a live prophylactic or therapeutic vaccine against infection by said *Francisella* species.

2. A strain according to claim 1 wherein said gene is inactivated by complete or partial deletion mutation or by insertional mutation.

3. A strain according to claim 1 or 2 which is a strain of *Francisella tularensis*.

4. A strain according to claim 3 which is a strain of *Francisella tularensis* subspecies *tularensis*.

5. A strain according to claim 3 which is a strain of *Francisella tularensis* subspecies *novicida*.

6. A pharmaceutical composition comprising a live strain of *Francisella* species wherein the purF or purD gene, has been inactivated, and which is able to produce a protective immune response in an animal, in combination with a pharmaceutically acceptable carrier.

7. A composition according to clam 6 wherein said gene is inactivated by complete or partial deletion mutation or by insertional mutation.

8. A pharmaceutical composition according to claim 6 or 7 wherein the strain is a strain of *Francisella tularensis*.

9. A pharmaceutical composition according to claim 7 wherein the strain is a strain of *Francisella tularensis* subspecies *tularensis* or a strain of *Francisella tularensis* subspecies *novicida*.

10. The use of a strain of *Francisella* species wherein the purF or purD gene has been inactivated, and which is able to produce a protective immune response in an animal, in the preparation of a live prophylactic or therapeutic vaccine against infection by said *Francisella* species.

11. The use according to claim 10 wherein said gene is inactivated by complete or partial deletion mutation or by insertional mutation.

12. The use according to claim 10 or 11 wherein the strain is a strain of *Francisella tularensis*.

13. The use according to claim 12 wherein the strain is a strain of *Francisella tularensis* subspecies *tularenis* or a strain of *Francisella tularensis* subspecies *novicida*.

14. A method for preparing a strain according to any one of claims 1 to 5, which comprises transforming a strain of *Francisella* species so as to inactivate said gene using cryotransformation.

15. A strain of Francisella tularensis subspecies tularensis wherein a gene that encodes a *purF* gene has been inactivated by complete or partial deletion mutation.

## Patentansprüche

1. Stamm einer *Francisella*-Art, in dem das purF- oder purD-Gen inaktiviert worden ist und der imstande ist, eine schützende Immunantwort in einem Tier hervorzurufen, zur Verwendung als prophylaktisches oder therapeutisches Lebendvakzin gegen eine Infektion durch diese *Francisella*-Art.

2. Stamm nach Anspruch 1, wobei das Gen durch vollständige oder partielle Deletionsmutation oder durch Insertionsmutation inaktiviert ist.

3. Stamm nach Anspruch 1 oder 2, der ein Stamm von *Francisella tularensis* ist.

4. Stamm nach Anspruch 3, der ein Stamm von *Francisella tularensis* Subspezies *tularensis* ist.

5. Stamm nach Anspruch 3, der ein Stamm von *Francisella tularensis* Subspezies *novicida* ist.

6. Pharmazeutische Zusammensetzung, die einen lebenden Stamm einer *Francisella*-Art, in dem das purF- oder purD-Gen inaktiviert worden ist und der imstande ist, eine schützende Immunantwort in einem Tier hervorzurufen, in Kombination mit einem pharmazeutisch akzeptablen Träger umfasst.

7. Zusammensetzung nach Anspruch 6, wobei das Gen durch vollständige oder partielle Deletionsmutation oder durch Insertionsmutation inaktiviert ist.

8. Pharmazeutische Zusammensetzung nach Anspruch 6 oder 7, wobei der Stamm ein Stamm von *Francisella tularensis* ist.

9. Pharmazeutische Zusammensetzung nach Anspruch 7, wobei der Stamm ein Stamm von *Francisella tularensis* Subspezies *tularensis* oder ein Stamm von *Francisella tularensis* Subspezies *novicida* ist.

10. Verwendung eines Stammes einer *Francisella*-Art, in dem das purF- oder purD-Gen inaktiviert worden ist und der imstande ist, eine schützende Immunantwort in einem Tier hervorzurufen, für die Herstellung eines prophylaktischen oder therapeutischen Lebendvakzins gegen eine Infektion durch diese *Francisella*-Art.

11. Verwendung nach Anspruch 10, wobei das Gen durch vollständige oder partielle Deletionsmutation oder durch Insertionsmutation inaktiviert ist.

12. Verwendung nach Anspruch 10 oder 11, wobei der Stamm ein Stamm von *Francisella tularensis* ist.

13. Verwendung nach Anspruch 12, wobei der Stamm ein Stamm von *Francisella tularensis* Subspezies *tularensis* oder ein Stamm von *Francisella tularensis* Subspezies *novicida* ist.

14. Verfahren zur Herstellung eines Stammes nach einem der Ansprüche 1 bis 5, das das Transformieren eines Stammes einer *Francisella*-Art mit dem Ziel der Inaktivierung des Gens unter Verwendung der Kryotransformation umfasst.

15. Stamm von *Francisella tularensis* Subspezies *tularensis*, in dem ein Gen, das ein *purF*-Gen codiert, durch vollständige oder partielle Deletionsmutation inaktiviert worden ist.

## Revendications

1. Souche de l'espèce *Francisella* dans laquelle le gène purF ou purD a été inactivé, et qui est capable de produire une réponse immunitaire de protection chez un animal, destinée à une utilisation en tant que vaccin vivant prophylactique ou thérapeutique contre une infection par ladite espèce *Francisella*.

2. Souche selon la revendication 1, dans laquelle ledit gène est inactivé par mutation par délétion complète ou partielle ou par mutation par insertion.

3. Souche selon la revendication 1 ou 2, qui est une souche de *Francisella tularensis*.

4. Souche selon la revendication 3, qui est une souche de *Francisella tularensis* sous-espèce *tularensis*.

5. Souche selon la revendication 3, qui est une souche de *Francisella tularensis* sous-espèce *novicida*.

6. Composition pharmaceutique comprenant une souche de l'espèce *Francisella* vivante dans laquelle le gène purF ou purD a été inactivé, et qui est capable de produire une réponse immunitaire de protection chez un animal, en combinaison avec un support pharmaceutiquement acceptable.

7. Composition selon la revendication 6, dans laquelle ledit gène est inactivé par mutation par délétion complète ou partielle ou par mutation par insertion.

8. Composition pharmaceutique selon la revendication 6 ou 7, dans laquelle la souche est une souche de *Francisella tularensis*.

9. Composition pharmaceutique selon la revendication 7, dans laquelle la souche est une souche de *Francisella tularensis* sous-espèce *tularensis* ou une souche de *Francisella tularensis* sous-espèce *novicida*.

10. Utilisation d'une souche de l'espèce *Francisella,* dans laquelle le gène purF ou purD a été inactivé, et qui est capable de produire une réponse immunitaire de protection chez un animal, dans la préparation d'un vaccin vivant prophylactique ou thérapeutique contre une infection par ladite espèce *Francisella.*

11. Utilisation selon la revendication 10, dans laquelle ledit gène est inactivé par mutation par délétion complète ou partielle ou par mutation par insertion.

12. Utilisation selon la revendication 10 ou 11, dans laquelle la souche est une souche de *Francisella tularensis*.

13. Utilisation selon la revendication 12, dans laquelle la souche est une souche de *Francisella tularensis* sous-espèce *tularensis* ou une souche de *Francisella tularensis* sous-espèce *novicida*.

14. Procédé de préparation d'une souche selon l'une quelconque des revendications 1 à 5, qui comprend la transformation d'une souche de l'espèce *Francisella* de manière à inactiver ledit gène en utilisant la cryotransformation.

15. Souche de *Francisella tularensis* sous-espèce *tularensis*, dans laquelle un gène qui code pour un gène purF a été inactivé par mutation par délétion complète ou partielle.


### SEQUENCE LISTING

<110> The Secretary of State for Defence
   Michell, Stephen L
   Oyston, Petra CF
   Quarry, Janine E
   Titball, Richard W
<120> Live vaccine strain
<130> CPG/P/222/WOD
<150> GB 0307089.3
   <151> 2003-03-27
<160> 10
<170> PatentIn version 3.1
<210> 1
   <211> 30
   <212> DNA
   <213> Artificial sequence
<220>
   <223> Primer CAM BamF
<400> 1
   cgcggatccg taagaggttc caactttcac 30
<210> 2
   <211> 33
   <212> DNA
   <213> Artificial sequence
<220>
   <223> Primer CAM BamR
<400> 2
   cgcggatcct tacgccccgc cctgccactc atc 33
<210> 3
   <211> 28
   <212> DNA
   <213> Artificial sequence
<220>
   <223> Primer sacBFor
<400> 3
   cgatctcaag aagcagaccg ctaacaca 28
<210> 4
   <211> 28
   <212> DNA
   <213> Artificial sequence
<220>
   <223> Primer sacBRev
<400> 4
   cgagctcata gttcatatgg gattcacc 28
<210> 5
   <211> 27
   <212> DNA
   <213> Artificial sequence
<220>
   <223> Primer screen F
<400> 5
   gtagaattcg taggtgtggt tggttag 27
<210> 6
   <211> 27
   <212> DNA
   <213> Artificial sequence
<220>
   <223> Primer screen R
<400> 6
   gtaacttgct gtcctgaata gtcttga 27
<210> 7
   <211> 30
   <212> DNA
   <213> Artificial sequence
<220>
   <223> Primer GuaA2For
<400> 7
   tgctctagat atagctattg ccgtaggaat 30
<210> 8
   <211> 30
   <212> DNA
   <213> Artificial sequence
<220>
   <223> Primer GuaABg1R
<400> 8
   cgtgcaagat ctgccattta gcattctcta 30
<210> 9
   <211> 27
   <212> DNA
   <213> Artificial sequence
<220>
   <223> Primer GuaA3Rev
<400> 9
   cgagctccag cgccaatacc agcacca 27
<210> 10
   <211> 30
   <212> DNA
   <213> Artificial sequence
<220>
   <223> Primer GuaAB1F
<400> 10
   gcacgtagat ctagcgtaga tatgagtatg 30